# EUROPÄISCHE PATENTANMELDUNG

(11) **EP 2 910 541 A1**
(43) Veröffentlichungstag der Anmeldung: **26.08.2015**
(21) Anmeldenummer: 15153246.2
(22) Anmeldetag: 30.01.2015
(51) Int. Cl.: C07C 45/28, C07C 45/65, C07C 5/32, C07C 49/403, C07C 49/385, C07C 49/08, C07C 47/02, C07C 11/02, C07C 13/20, C07C 13/12

(54) **Verfahren zur photokatalytischen akzeptorfreien Dehydrierung von Alkanen und Alkoholen**

(30) Priorität: 25.02.2014 DE 102014203341
(71) Anmelder: Evonik Degussa GmbH, 45128 Essen (DE)
(72) Erfinder: Franke, Robert, 45772 Marl (DE); Julis, Jennifer, 40479 Düsseldorf (DE); Fridag, Dirk, 45721 Haltern am See (DE); Dyballa, Katrin Marie, 45657 Recklinghausen (DE); Weding, Nico, 58511 Lüdenscheid (DE); Dutta Chowdhury, Abhishek, 743222 West Bengal (IN); Beller, Matthias, 18211 Nienhagen (DE); Jackstell, Ralf, 27478 Cuxhaven Altenwalde (DE); Fleischer, Ivana, 93057 Regensburg (DE); Percia, Beatrice, 624802 Vadamadurai, TamilNadu (IN)

(57) **Zusammenfassung**

Die Erfindung betrifft ein Verfahren zur photokatalytischen akzeptorfreien Dehydrierung von Alkanen und Alkoholen, bei dem ein Alkan oder ein Alkohol in Gegenwart einer Rhodiumkomplexverbindung, die organische Phosphor(III)-Verbindungen als Liganden enthält, als Katalysator, sowie in Gegenwart von mindestens einer Lewis Base, bestrahlt wird.

## Beschreibung

Die Erfindung betrifft ein Verfahren zur photokatalytischen akzeptorfreien Dehydrierung von Alkanen und Alkoholen.

Alkene werden als die wichtigsten und vielseitigsten Rohstoffe in der Chemischen Industrie angesehen. Im Gegensatz zu den Alkanen sind sie jedoch weit weniger verfügbar. Deshalb hat die direkte katalytische Dehydrierung von Alkanen zu Alkenen eine große Aufmerksamkeit als einer der effizientesten und ökonomischsten Routen zur Alkenbildung in Forschungsaktivitäten erfahren [Choi, J., Roy MacArthur, A. H.; Brookhart, M.; Goldman, A. S. Chem. Rev. 2011, 111, 1761-1779].

Die akzeptorfreie Alkandehydrierung wird aufgrund der Einfachheit und Atomökonomie als ein idealer Prozess betrachtet. Solch eine atomökonomische und akzeptorfreie Dehydrierung kann unter photokatalytischen Bedingungen erreicht werden. Jedoch ist diese Reaktion durch lange Reaktionszeiten, Katalysatordeaktivierung und geringere Reaktivität im Vergleich zur katalytischen Dehydrierung/Transferhydrierungsreaktion limitiert. Von Nomura, K.; Saito, Y. J. Chem. Soc., Chem. Commun.1988, 161-162 ist bekannt, dass der Katalysator Rh(PMe₃)₂(CO)Cl sowohl in der akzeptorfreien photokatalytische Dehydrierung als auch in der thermochemischen Transferhydrierung aktiv ist, jedoch nur unter Wasserstoffdruck, was dessen Anwendung zur Synthese von Alkenen einschränkt.

Um Alkandehydrierung bei relativ niedriger Temperatur unter homogenen Bedingungen zu realisieren, werden normalerweise Opferolefine (Akzeptoren) eingesetzt. Allgemein wird in der Literatur eine effiziente Alkantransferhydrierung nur mit einem großen Überschuss eines Opferolefins (bis zu 20-fachem Überschuss) erreicht, was eine potentielle sinnvolle Anwendbarkeit einschränkt. Auch sind die technisch relevanten Turnoverzahlen (TON) dieser Methoden normalerweise mit ca. 1000 bei vernünftigen Reaktionszeiten limitiert. Turnoverzahlen (TON) >1000 werden erst bei langen Reaktionszeiten von mehreren Tagen erreicht. Obwohl es kontinuierlich verschiedene Anstrengungen zur homogenen katalytischen Alkandehydrierungen in den letzten 30 Jahren gab, ist immer noch ein erheblicher Bedarf für signifikante Verbesserungen gegeben, speziell für die akzeptorfreie atomökonomische Alkandehydrierung. Derzeit erfolgreiche Alkandehydrierungen hängen hauptsächlich vom Verhalten verschiedener spezieller anspruchsvoller Pincerliganden und deren thermischen Stabilität (auch ihrer Metallkomplexe) ab. Um die hohe Endothermie der Alkandehydrierung zu überwinden wurden normalerweise hohe Reaktionstemperaturen von bis zu 250 °C oder lange Reaktionszeiten von bis zu 3 Tagen angewandt. Deshalb ist die Reaktivität streng durch die thermische Stabilität des Katalysators bestimmt. Ein weiteres Problem stellt die Inhibierung der Reaktion durch das Olefin dar, welches entweder als Opferolefin oder als Produkt präsent ist, speziell bei längeren Reaktionszeiten.

Der Erfindung lag deshalb die Aufgabe zugrunde ein Verfahren zur akzeptorfreien Alkandehydrierung zu entwickeln, das Olefine als Akzeptoren vermeidet und eine atomökonomische Alkandehydrierung gestattet, wobei lange Reaktionszeiten, Katalysatordeaktivierung und geringe Reaktivität vermieden werden sollen.

Die Aufgabe wird gelöst durch ein Verfahren nach Anspruch 1.

Verfahren zur photokatalytischen akzeptorfreien Dehydrierung von Alkanen und Alkoholen, dadurch gekennzeichnet, dass ein Alkan oder ein Alkohol in Gegenwart einer Rhodiumkomplexverbindung, die organische Phosphor(III)-Verbindungen als Liganden enthält, als Katalysator, sowie in Gegenwart von mindestens einer Lewis Base, bestrahlt wird.

Die Erfindung beruht dabei auf der überraschenden Erkenntnis, dass eine photokatalytische akzeptorfreie Dehydrierung von Alkanen in Gegenwart von mindestens einer Lewis Base, ein effizientes atomökonomisches photokatalytisches Verfahren ermöglicht, womit Alkane als auch Alkohole, mit hohen Turnoverzahlen in einem breiten Substratbereich dehydriert werden können.

Im Gegensatz zur thermischen Dehydrierung [Balsells, R. E.; Frasca, A. R. Tetrahedron 1982, 38, 245-255] von Alkoholen sind noch keine Untersuchungen zu photokatalytischen Dehydrierungen von Alkoholen literaturbekannt.

In einer Variante des Verfahrens wird dieses zur photokatalytischen akzeptorfreien Dehydrierung von Alkoholen eingesetzt.

In einer anderen Variante des Verfahrens wird dieses zur photokatalytischen akzeptorfreien Dehydrierung von Alkanen eingesetzt.

In einer anderen Variante des Verfahrens weisen das Alkan oder der Alkohol eine Kohlenstoffkettenlänge von 2 bis 30 C-Atome auf. Vorzugsweise weisen das Alkan oder der Alkohol eine Kohlenstoffkettenlänge von 2 bis 12 C-Atome auf, besonders bevorzugt eine Kohlenstoffkettenlänge von 4 bis 10 C-Atome.

Alkane und Alkohole weisen bevorzugt eine Kohlenstofflänge von 2 bis 30 C-Atomen auf, Isoformen sowie cyclische Verbindungen sind eingeschlossen. Die Verbindungen können substituiert sein.

Spezielle Beispiele für die zu verwendenden Alkane, Cycloalkane, Alkohole und Cycloalkohole sind im Folgenden genannt.

Als Alkane der allgemeinen Formel CₙH₂ₙ₊₂ (n = 2 bis 30) können beispielsweise Ethan, Propane, Butane, Pentane, Hexane, Heptane, Octane, Nonane, Decane, Undecane, Dodecane und dergleichen im erfindungsgemäßen Verfahren eingesetzt werden. Cycloalkane, die durch die allgemeine Formel CₙH₂ₙ (n = 4 bis 30) dargestellt werden können, sind z.B. Cyclopentan, Cyclohexan, Cycloheptan, Cyclooctan, Cyclododecan, sowie alkylsbstituierte Alkane wie z. B. Methylcyclohexan und dergleichen.

Die Alkohole und Cycloalkohole leiten sich von den Alkanen und Cycloalkanen ab. Im Wesentlichen sind Cycloalkohole reaktivere Substrate im Vergleich zu linearen oder substituierten acyclischen Alkoholen unter ähnlichen Verfahrensbedingungen. Bevorzugt sind lineare und verzweigte aliphatische Alkohole der allgemeinen Formel CₙH₂ₙ₊₁OH (n= 2 bis 12) und cycloaliphatische Alkohole der allgemeinen Formel CₙH₂ₙ₋₁OH (n= 5 bis 12). Beispielsweise seien genannt: Propanole, Butanole, Pentanole, Hexanole, Cyclohexanol, Methylcyclohexanole, Heptanole, Octanole, Cyclooctanol, Nonanole, Decanole, Undecanole und Dodecanole, Im erfindungsgemäßen Verfahren können auch substituierte Alkane/Cycloalkane und substituierte Alkohole/Cycloalkohole eingesetzt werden. Der Substituent kann jeweils mindestens eine Alkylgruppe oder aromatische Gruppe sein, die eine Carboxylgruppe, Estergruppe, Halogengruppe, Nitrogruppe oder Methoxygruppe aufweisen kann. Aber auch eine Carboxylgruppe, Estergruppe, Halogengruppe, Nitrogruppe oder Methoxygruppe kommen als Substituenten in Frage. Alkylsubstituenten haben bevorzugt 1 bis 6 C-Atome.

Als bevorzugte Beispiele für die Alkane werden n-Butan, n-Pentan, n-Hexan, 2-Methylpentan, n-Heptan, n-Octan, n-Nonan, n-Decan, n-Dodecan genannt. Bevorzugte Beispiele für die Cycloalkane sind Methylcyclohexan, Adamantan, cis-Decalin, trans-Decalin, Cyclohexan und dergleichen. Die Cycloalkane umfassen einen kondensierten Ring, wobei auch ein aromatischer Ring und ein Cycloalkanring in ortho-Position kondensiert sein können. Beispiele hierfür sind Indan, Tetralin, Fluoren und dergleichen. Bevorzugte Alkohole/Cycloalkohole sind beispielsweise n-Propanol, i-Propanol, n-Butanol, i-Butanol, n-Pentanol, n-Hexanol, Cyclohexanol, n-Heptanol, n-Octanol, Cyclooctanol, n-Nonanol und dergleichen.

In einer anderen Variante des Verfahrens ist die Lewis Base ein organisches Amin.

In einer anderen Variante des Verfahrens ist die Lewis Base ein heterozyklisches Amin.

In einer anderen Variante des Verfahrens ist die Lewis Base ein Bipyridin.

In einer anderen Variante des Verfahrens wird als Lewis Base 2,2-Bipyridin oder 4,4-Bipyridin verwendet.

In einer anderen Variante des Verfahrens wird als Lewis Base Bathocuproin oder Phenanthrolin verwendet.

In einer Variante des Verfahrens wird die Reaktion in Gegenwart von CO₂ durchgeführt wird.

Es wurde des Weiteren gefunden, dass in Gegenwart von CO₂ die Reaktionen zur Dehydrierung besser verlaufen mit höheren Ausbeuten bei längerer Reaktionsführung.

In einer Variante des Verfahrens erfolgt die Bestrahlung mit einer Lichtquelle, die Licht über einen Wellenängenbereich von 320 nm bis 500 nm ausstrahlt.

Auch der Einfluss der Wellenlänge der verwendeten Lichtquelle wurde untersucht und es wurde gefunden, dass eine Bestrahlung bevorzugt mit Licht im Wellenlängenbereich von λ = 320 nm bis 500 nm erfolgt.

In einer Variante des Verfahrens wird die Dehydrierung bei einer Temperatur von 45 °C bis 120 °C durchgeführt.

Die erfindungsgemäße Dehydrierung findet weiterhin besonders optimal bei einer Temperatur von vorzugsweise 45 °C bis 120 °C statt. Eine Temperatur von 80 °C bis 95 °C ist besonders bevorzugt. Die besten Ausbeuten werden bei einer Reaktionstemperatur von 85 °C bis 90 °C erzielt.

Darüber hinaus ist die Verwendung eines Glasreaktors oder eines Metallreaktors, der Glas aufweist, im erfindungsgemäßen Verfahren besonders effektiv. Einwandige Glasreaktoren, vorzugsweise mit einer Wandstärke von 1,0 bis 3,0 mm, sind besonders geeignet. Interessanterweise findet kaum eine Reaktion statt, wenn man lediglich einen geeigneten Metallreaktor mit Lichteingang verwendet. Überraschend hat die Gegenwart von Glas einen bemerkenswerten Einfluss auf die erfindungsgemäße Reaktion, wobei neben dem Material auch die Wandstärke des verwendeten Glasgefäßes bedeutsam sein kann. So kann die Reaktion zwar mit Glasgefäßen unterschiedlicher Wandstärke durchgeführt werden, eine Wandstärke von 1,2 mm bis 1,8 mm hat sich als besonders optimal erwiesen.

In einer Variante des Verfahrens weist die Glasscheibe eine Dicke von 1,2 mm bis 3,0 mm auf.

Vorteilhafterweise werden deshalb dünnwandige Glasgefäße im erfindungsgemäßen Verfahren eingesetzt, da die Transmission in diesen erheblich größer ist und deshalb mehr Energie zur Alkandehydrierung zur Verfügung steht. Dieser starke Einfluss der Anwesenheit von Glas und der Wandstärke der Reaktionsgefäße wurde bis jetzt noch nicht in photokatalytischen Reaktionen nachgewiesen.

In einer Variante des Verfahrens werden einwandige Glasreaktoren eingesetzt.

In einer Variante des Verfahrens wird ein Rhodiumkomplex der allgemeinen Formel (I) eingesetzt:

Rh(L¹)₂(CO)X (**I**),

worin L¹ = P(C₁-C₅Alkyl)₃ ist,
und X für ein Anion steht ausgewählt aus: Chlorid, Bromid, Acetat.

In einer Variante des Verfahrens ist L¹ in (I) = PMe₃ oder P*t*Bu₃.

In einer Variante des Verfahrens steht X in (I) für Chlorid.

In einer Variante des Verfahrens wird ein Rhodiumkomplex der allgemeinen Formel **(II)** eingesetzt:

Rh₂(L²)₂(CO)₂(X)₂ **(II)**,

worin L² = P(PH)₂(CH₂)ₙ(Ph₂)P ist mit n = 1 bis 3,
und X für ein Anion steht ausgewählt aus: Chlorid, Bromid, Acetat.

In einer Variante des Verfahrens ist L² in **(II)** = P(PH)₂(CH₂)(Ph₂)P.

In einer Variante des Verfahrens steht in **(II)** X für Chlorid steht.

In einer Variante des Verfahrens wird der gebildete Wasserstoff aus dem Reaktionsgemisch entfernt.

Das vorliegende Verfahren gestattet eine atomökonomische Alkandehydrierung bei geringen Reaktionszeiten, wobei der Katalysator stabil bleibt. So wurde z.B. mit n-Octan als Modellsubstrat in Abwesenheit von Additiven eine Ausbeute von lediglich 3,5 % an Octenen d.h. eine TON von 333 in 3 h bzw. eine Turnoverfrequeunz von 111 h⁻¹ erzielt.

In Gegenwart der Lewis Base 4,4'-Bipyridin wurde dagegen in einem Glasgefäß mit der Wandstärke von 1,2 mm im erfindungsgemäßen Verfahren ca. 20% Ausbeute mit einer TON von 1518 und einer TOF von 217 h⁻¹ innerhalb von 7 Stunden erreicht. Bei der Verwendung anderer Substrate wie Cyclooctan werden ca. 40% an cis-Cycloocten mit einer TON = 2883 innerhalb von 7 h erzielt. Analog werden z.B. mit Methylcyclohexan 16.5% Ausbeute mit einer TON von 1200 erzielt.

Um eine noch effektivere Reaktion durchzuführen, kann der gebildete Wasserstoff vorteilhafter Weise aus der Reaktionslösung entfernt werden. Das erfolgt z.B. durch eine hohe Rührgeschwindigkeit und konstantem Gasstrom (z.B. Argon). Durch diese Reaktionsführung können die erzielten TON weiter erhöht werden und sind somit wesentlich höher als in der Literatur beschriebene Systeme.

Die Bestrahlung erfolgt bevorzugt mit Licht, vorzugsweise über einen Wellenlängebereich von λ = 320 nm bis 500 nm. Die Dehydrierung erfolgt weiterhin besonders optimal bei einer Temperatur von vorzugsweise 45 °C bis 120 °C, vorzugsweise bei einer Temperatur von 80 °C bis 95°C. Insbesondere ist eine Reaktionstemperatur von 85 °C bis 90°C effektiv. Weiterhin ist die Verwendung eines Glasreaktors oder eines Metallreaktors, der Glas aufweist, im erfindungsgemäßen Verfahren besonders effektiv. Einwandige Glasreaktoren, vorzugsweise mit einer Glasdicke von 1,0 bis 3,0 mm, sind besonders geeignet. Der verwendete Rhodiumkomplex ist vorzugsweise einer der allgemeinen Formel (I), der oben genannt ist, insbesondere mit L¹ = PMe₃ oder PBu₃ und X = Cl. In der Regel ist der bevorzugte Katalysator Rh(PMe₃)₂(CO)Cl.

Im Folgenden wird die Erfindung an Ausführungsbeispielen näher erläutert.

### Ausführungsbeispiele

**Allgemeine Reaktionsbedingungen:** Alle synthetischen Operationen wurden unter Argon in getrockneten Duranborosilicatglasgefäßen mit geeigneten Schlenktechniken durchgeführt. Der Katalysator Rh(PMe₃)₂(CO)Cl wurde analog einer Literaturvorschrift hergestellt. [Bridgewater, J. S.; Netzel, T. L.; Schoonover, J. R.; Massick, S. M.; Ford, P. C. Inorg. Chem. 2001, 40, 1466-1476]. Die Produkte wurden gegen eine Vergleichsprobe analysiert und die Ausbeute mittels Gaschromatographie (Agilent 6890N network GC System mit einer (60m×250µm×0.25µm) DB Wax Säule und Isooctan als internen Standard (0,2 ml, 1,2 mmol) nach einer Verdünnung mit Aceton. Die Responsfaktoren jedes Produktes wurden mittels 'Multiple Point Internal Standard GC Quantitation Method' gegen Isooctan bestimmt. Für alle Analysen wurden folgende Bedingungen gewählt: N₂ als Trägergas, Einlasstemperatur: 250 °C, Einlassdruck: 104.4 KPa, Injektionsvolumen: 1,0 µl, Splitverhältnis: 100:1, Splitflow: 80,0 ml/min, Flussrate: 0,8 ml/min bis 20 min die dann auf 2,8 ml/min 1.0 ml/min² erhöht wurde, Temperatur: 35 °C bis 20 min, anschließend erhöht auf 40 °C/min bis 200°C und dann 15 min bei 200 °C gehalten. Detektortemperatur: 250 °C, Wasserstofffluss: 30 ml/min, Luft: 300 ml/min, Stickstroffstrom: 25 ml/min.

Allgemeine Arbeitsvorschrift 1: Das entsprechende Glasgefäß versehen mit einem Rückflusskühler und Magnetrührer wird unter Argon mit 0,004 mmol des Katalysators Rh(PMe₃)₂(CO)Cl und 0,02 mmol des entsprechenden Additives befüllt. Sehr genaues Arbeiten unter Argon als Schutzgas ist notwendig, da der Katalysator sehr leicht in Gegenwart von Luftsauerstoff und Licht deaktiviert wird. Es werden anschließend 30 mmol Substrat zugesetzt und ein Argonstrom angelegt. Die Rührgeschwindigkeit wird auf 1000 min⁻¹ eingestellt und das Glasgefäß mit Aluminiumfolie umschlossen. Dann wird die verwendete Lichtquelle Lumatec Superlite 400, die Licht über einen Wellenlängenbereich von 320 nm bis 500 nm ausstrahlt, angeschaltet. Nach der Reaktion wird die Lichtquelle ausgeschaltet, die Reaktionslösung abgekühlt und die Ausbeute mittels Gaschromatografie und Isooctan als internem Standard bestimmt. Die Turnoverzahlen (TON) in den Tabellen werden mit [mmol Produkt]/[mmol Katalysator] berechnet.

Allgemeine Arbeitsvorschrift 2: Das entsprechende Glasgefäß versehen mit einem Rückflusskühler und Magnetrührer wird unter Argon mit 0,004 mmol des Katalysators Rh(PMe₃)₂(CO)Cl und 0,02 mmol des entsprechenden Additives befüllt. Sehr genaues Arbeiten unter Argon als Schutzgas ist notwendig, da der Katalysator sehr leicht in Gegenwart von Luftsauerstoff und Licht deaktiviert wird. Es werden anschließend 30 mmol Substrat zugesetzt und ein Argonstrom angelegt. Die Rührgeschwindigkeit wird auf 1000 min⁻¹ eingestellt und das Glasgefäß mit Aluminiumfolie umschlossen. Es wird mittels einer Metallkapillare ein CO₂-Strom durch die Lösung geleitet. Dann wird die verwendete Lichtquelle Lumatec Superlite 400 angeschaltet. Nach der Reaktion wird die Lichtquelle ausgeschaltet, der CO₂-Strom beendet, die Reaktionslösung abgekühlt und die Ausbeute mittels Gaschromatografie und Isooctan als internem Standard bestimmt. Die Turnoverzahlen (TON) in den Tabellen werden mit [mmol Produkt]/[mmol Katalysator] berechnet.

### Beispiel 1

### Umsatz von erfindungsgemäß verwendeten Additiven zur Dehydrierung von n-Octan

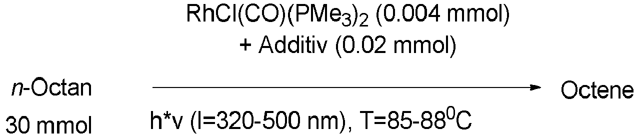

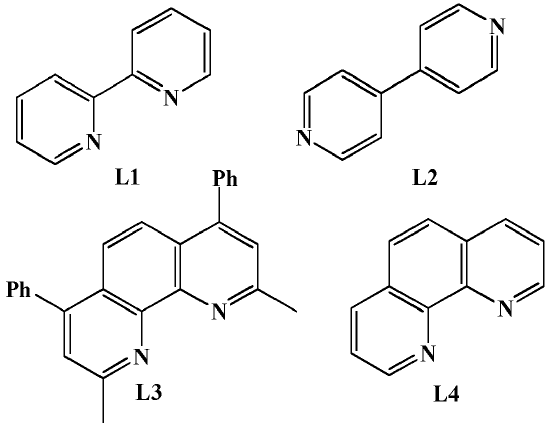

### Beispiel 1.1:

Ein doppelwandiger Dreihalsphotoreaktor mit einer Wandstärke von 2,3 mm versehen mit einem Rückflusskühler und Magnetrührer wird mit 1,3 mg Rh(PMe₃)₂(CO)Cl (0,004 mmol) und 3.1 mg 2,2'-Bipyridin (L1, 0,02 mmol) bestückt. Anschließend wird das Reaktionsgefäß dreimal evakuiert und mit Argon befüllt, um inerte Bedingungen zu realisieren. Es werden anschließend 5 ml n-Octan (30 mmol) zugegeben. Es wird ein Argonstrom angelegt, um gebildeten Wasserstoff zu entfernen. Der Reaktor wird mit Aluminiumfolie umschlossen und die Lichtquelle mit einer Wellenlänge 320 nm bis 500 nm Wellenlänge (Lumatec Superlite 400) angeschaltet. Es wird drei Stunden bei 1000 min⁻¹ gerührt. Nach der Reaktion wird die Lichtquelle ausgeschaltet und die Reaktionslösung abgekühlt. Die Ausbeute wird mittels Gaschromatografie und Isooctan als internem Standard bestimmt.

### Beispiel 1.2:

In Analogie zu Beispiel 1.1 werden 3,1 mg 4,4'-Bipyridin (L2, 0,02 mmol) als Additiv verwendet.

### Beispiel 1.3:

In Analogie zu Beispiel 1.1 werden 3,1 mg 4,4'-Bipyridin (L2, 0,02 mmol) als Additiv verwendet und es wird fünf Stunden bei 1000 min⁻¹ gerührt.

### Beispiel 1.4:

In Analogie zu Beispiel 1.1 werden 7,2 mg Bathocuproin (L3, 0,02 mmol) als Additiv verwendet.

### Beispiel 1.5:

In Analogie zu Beispiel 1.1 werden 3,6 mg Phenanthrolin (L4, 0,02 mmol) als Additiv verwendet. Ausbeute und Umsatzraten sind in Tabelle 1 dargestellt.

**Tabelle 1**

| Nr. | Additiv | Zeit (h) | Ausbeute (%) | TON | TOF (h⁻¹) |
|---|---|---|---|---|---|
| 1.1 | 2,2'-Bipyridin (L1) | 3 | 4.8 | 419 | 140 |
| 1.2 | 4,4'-Bipyridin (L2) | 3 | 4.2 | 411 | 137 |
| 1.3 | 4,4'-Bipyridin (L2) | 5 | 5.4 | 489 | 99 |
| 1.4 | Bathocuproin (L3) | 3 | 3.1 | 310 | 104 |
| 1.5 | Phenanthrolin (L4) | 3 | 2.1 | 155 | 52 |

### Beispiel 2

Reaktion unter Verwendung verschiedener Glasreaktoren mit n-Octan als Substrat und dem Additiv L2 (4,4'-Bipyridin)

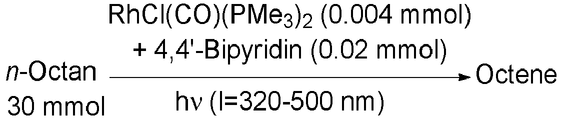

Ein Schlenkgefäß mit Wandstärken von 1,2 mm; 1,6 mm, 1,8 mm und 3 mm sowie ein doppelwandiger Dreihalsphotoreaktor und verspiegelter doppelwandiger Dreihalsphotoreaktor mit einer Wandstärke von 2,3 mm versehen mit einem Rückflusskühler und Magnetrührer werden jeweils mit 1,3 mg Rh(PMe₃)₂(CO)Cl (0,004 mmol) und 3,1 mg 4,4'-Bipyridin (L2, 0,02 mmol) bestückt. Anschließend wird das jeweilige Reaktionsgefäß dreimal evakuiert und mit Argon befüllt, um inerte Bedingungen zu realisieren. Es werden anschließend 5 ml n-Octan (30 mmol) zugegeben. Es wird ein Argonstrom angelegt, um gebildeten Wasserstoff zu entfernen. Die Reaktoren werden mit Aluminiumfolie umschlossen und die Lichtquelle mit einer Wellenlänge 320-500 nm Wellenlänge (Lumatec Superlite 400) angeschaltet. Es wird sieben bzw. fünf Stunden bei 1000 min⁻¹ gerührt. Nach der Reaktion wird die Lichtquelle ausgeschaltet und die Reaktionslösung abgekühlt. Die Ausbeute wird mittels Gaschromatografie und Isooctan als internem Standard bestimmt.

Tabelle 2 zeigt Bedingungen, Ausbeuten und Umsatzraten.

**Tabelle 2**

| Nr.^{a} | Glasgefäß (Wandstärke,mm) | Zeit (h) | Ausbeute(%) | TON | TOF (h⁻¹) |
|---|---|---|---|---|---|
| 2.1 | Schlenkgefäß (1.2)^{b} | 7 | 19.7 | 1518 | 217 |
| 2.2 | Schlenkgefäß (1.2) | 5 | 16.7 | 1353 | 270 |
| 2.3 | Schlenkgefäß (1.6) | 5 | 14.8 | 1268 | 253 |
| 2.4 | Schlenkgefäß (1.8) | 5 | 12.3 | 1069 | 211 |
| 2.5 | Schlenkgefäß (3.0) | 5 | 9.2 | 746 | 150 |
| 2.6 | Photoreaktor (2.3) | 5 | 5.2 | 489 | 99 |
| 2.7 | Verspiegelter Photoreaktor (2.3) | 5 | 8.0 | 636 | 127 |

| | | | | | |
|---|---|---|---|---|---|
| ^{a} Im Photoreaktor: Licht, Glas, Argon, Lösung, im Schlenkgefäß: Licht, Glas, Lösung. ^{b} Zusammensetzung: 7% 1-Octen, 9% 2-Octen, 1% 3-Octen, 3% 4-Octen und Spuren von Dienen. | | | | | |

### Beispiel 3

Reaktion unter Verwendung verschiedener Glasreaktoren mit Cyclooctan als Substrat und dem Additiv L2 (4,4'-Bipyridin)

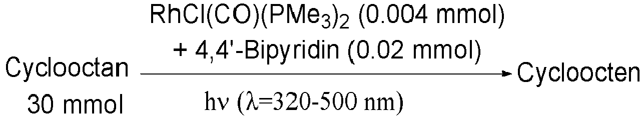

Die Reaktion erfolgte analog zu Beispiel 2 unter Einsatz von 4 mL Cyclooctan (30 mmol). Tabelle 3 zeigt Bedingungen, Ausbeuten und Umsatzraten.

**Tabelle 3**

| Eintrag^{a} | Glasgefäß (Wandstärke, mm) | Zeit (h) | Ausbeute (%) | TON | TOF (h⁻¹) |
|---|---|---|---|---|---|
| 3.1 | Schlenkgefäß (1.2) | 7 | 40 | 2883 | 411 |
| 3.2 | Schlenkgefäß (1.2) | 5 | 38.4 | 2747 | 549 |
| 3.3 | Schlenkgefäß (1.6) | 5 | 23.8 | 1741 | 348 |
| 3.4 | Schlenkgefäß (1.8) | 5 | 23.8 | 1741 | 348 |
| 3.5 | Schlenkgefäß (3.0) | 5 | 8 | 618 | 123 |
| 3.6 | Photoreaktor (2.3) | 5 | 5.2 | 460 | 92 |

| | | | | | |
|---|---|---|---|---|---|
| ^{a} Im Photoreaktor: Licht, Glas, Argon, Lösung, im Schlenkgefäß: Licht, Glas, Lösung. Geringe Spuren eines nicht identifizierten Produktes mit m/z=220 (via GCMS) und <1% für Eintrag 1. | | | | | |

### Beispiel 4

In Analogie zu Beispiel 2 und 3 werden weitere Substrate (jeweils 30 mmol) unter Verwendung eines 1,2 mm dicken einwandigen Schlenkgefäßes eingesetzt.

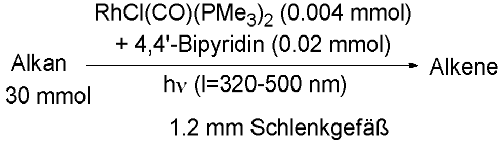

**Tabelle** 4 zeigt Substrate, Bedingungen, Ausbeuten und Umsatzraten.

**Tabelle 4**

| Eintrag | Alkan | Zeit (h) | Ausbeute(%) | TON | TOF (h⁻¹) |
|---|---|---|---|---|---|
| 4.1^{a} | Cyclohexan | 7 | 13 | 975 | 195 |
| 4.2 | | 5 | 12.1 | 951 | 191 |
| 4.3^{b} | Methylcyclohexan | 7 | 16.5 | 1200 | 171 |
| 4.4 | | 5 | 16.1 | 1150 | 171 |
| 4.5^{c} | n-Hexan | 7 | 6.5 | 462 | 66 |
| 4.6^{c,d} | | 5 | 5.6 | 414 | 83 |
| 4.7^{e} | 2-Methylpentan | 7 | 4.7 | 368 | 53 |
| 4.8^{e,f} | | 5 | 4.3 | 342 | 68 |
| 4.9 | n-Dodecan | 7 | 14.6 | 1100 | 220 |
| 4.10^{g} | Tetralin | 7 | 5 | 357 | 51 |
| 4.11 | Decalin (cis+trans) | 7 | 2.2 | 165 | 24 |
| 4.12 | Indolin | 7 | 9.3 | 708 | 101 |

| | | | | | |
|---|---|---|---|---|---|
| ^{a} Spuren eines unbekannten Produktes mit m/z=164 (via GCMS). ^{b} Zusammensetzung: 0.8% Methylenecyclohexan, 1.6% 1-Methyl-1-cyclohexen, 10% 1-Methyl-3-Cyclohexen, 4% 1-Methyl-4-cyclohexenund Spuren Cyclopenten. ^{c}Alkanverlust. ^{d}Zusammensetzung: 0.3% 1-Hexen, 4.8% 2-Hexen, 1.4% 3-Hexen. ⁶ signifikanter Alkanverlust. ^{f}Zusammensetzung: 3.4% 4-Methyl-1-penten, 1% 2-Methyl-1-penten, 0.3% 4-Methyl-2-penten & 2-Methyl-2-penten. ^{g}4% 1,2-Dihydronaphthalen, ∼1% 1,4-Dihydronaphthalen und Spuren Naphthalen. | | | | | |

### Beispiel 5

### Gleichzeitiger Umsatz von linearen und cyclischen Alkanen

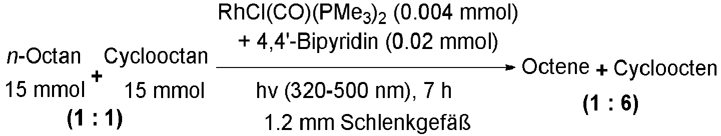

Ein Schlenkgefäß mit einer Wandstärke von 1,2 mm versehen mit einem Rückflusskühler und Magnetrührer wird mit 1,3 mg Rh(PMe₃)₂(CO)Cl (0.004 mmol) und 3,1 mg 4,4'-Bipyridin (L2, 0,02 mmol) bestückt. Anschließend wird das Reaktionsgefäß dreimal evakuiert und mit Argon befüllt, um inerte Bedingungen zu realisieren. Es werden anschließend 2,5 ml n-Octan (15 mmol) und 2 ml Cyclooctan (15 mmol) zugegeben. Es wird ein Argonstrom angelegt, um gebildeten Wasserstoff zu entfernen. Der Reaktor wird mit Aluminiumfolie umschlossen und die Lichtquelle mit einer Wellenlänge 320-500 nm Wellenlänge (Lumatec Superlite 400) angeschaltet. Es wird sieben Stunden bei 1000 min⁻¹ gerührt. Nach der Reaktion wird die Lichtquelle ausgeschaltet und die Reaktionslösung abgekühlt. Die Ausbeute wird mittels Gaschromatografie und Isooctan als internem Standard bestimmt und ist in Tabelle 5 dargestellt.

**Tabelle 5**

| Eintrag | Alken | Ausbeute (%) | TON | TOF (h⁻¹) |
|---|---|---|---|---|
| 5.1 | Octen | 3.0 | 200 | 29 |
| | Cycloocten | 35 | 1193 | 170 |

Zusammenfassend für die Alkandehydrierung ist festzustellen, dass Ausbeute bzw. Reaktivität der Substrate hauptsächlich von der Enthalpie der Dehydrierung bestimmt ist. Die höhere Ausbeute bei der Cyclooctandehydrierung (Beispiel 3.2) ist demnach mit der geringeren Enthalpie von 23,3 kcal/mol zu erklären während für das Cyclohexan mit einer wesentlich höheren Enthalpie von 28,2 kcal/mol eine geringere Ausbeute zu verzeichnen ist (Beispiel 4.2). Die Enthalpie für Methylcyclohexan liegt mit 26,5 kcal/mol genau dazwischen und auch die Ausbeute an dem entsprechenden Dehydrierprodukt (Beispiel 4.4). Die Enthalpie für lineare C6-C10 Alkane liegen zwischen 27 und 30 kcal/mol. Deshalb ist die Endothermie der entscheidende Faktor in den präsentierten Beispielen. Dies wird auch durch das obige Kontrollexperiment verifiziert in dem n-Octan und Cyclooctan im Gemisch 1:1 eingesetzt wurde. Tabelle 5 zeigt eindrucksvoll die Präferenz für die Dehydrierung des Cyclooctans.

### Beispiel 6

### Dehydrierung von Alkoholen

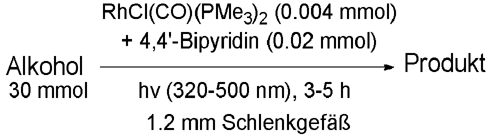

Ein Schlenkgefäß mit einer Wandstärke von 1,2 mm versehen mit einem Rückflusskühler und Magnetrührer wird mit 1,3 mg Rh(PMe₃)₂(CO)Cl (0.004 mmol) und 3.1 mg 4,4'-Bipyridin (L2, 0.02 mmol) bestückt. Anschließend wird das Reaktionsgefäß dreimal evakuiert und mit Argon befüllt, um inerte Bedingungen zu realisieren. Es werden anschließend 3,2 ml Cyclohexanol, 3,9 ml Cyclooctanol, 2,3 ml Isopropanol, 1-Hexanol, 1-Nonanol (jeweils 30 mmol) zugegeben. Es wird ein Argonstrom angelegt, um gebildeten Wasserstoff zu entfernen. Der Reaktor wird mit Aluminiumfolie umschlossen und die Lichtquelle mit einer Wellenlänge 320-500 nm Wellenlänge (Lumatec Superlite 400) angeschaltet. Es wird drei Stunden bei 1000 min⁻¹ gerührt. Nach der Reaktion wird die Lichtquelle ausgeschaltet und die Reaktionslösung abgekühlt. Die Ausbeute wird mittels Gaschromatografie gemäß obiger Reaktionsgleichung.

Tabelle 6 zeigt verwendete Alkoholsubstrate, Bedingungen, Ausbeuten und Umsatzraten.

**Tabelle 6**

| Eintrag | Substrat | Produkt | Zeit (h) | Ausbeute (%) | TON | TOF (h⁻¹) |
|---|---|---|---|---|---|---|
| 7.1 | Cyclohexanol | Cyclohexanon | 3 | 3.9 | 288 | 96 |
| 7.2 | Cyclooctanol | Cyclooctanon | 3 | 6.8 | 454 | 151 |
| 7.3 | Iso-propanol | Aceton | 3 | 2.5 | 189 | 38 |
| 7.4 | 1-Hexanol | Hexanal | 3 | 0.5 | 38 | 13 |
| 7.5 | 1-Nonanol | Nonanal | 3 | 1.3 | 101 | 34 |

### Beispiel 7

Reaktion unter Verwendung eines 1,2 mm dicken einwandigen Schlenkgefäßes mit n-Octan als Substrat und dem Additiv L2 (4,4'-Bipyridin) in Gegenwart von CO₂.

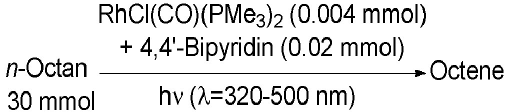

Ein Schlenkgefäß mit einer Wandstärke von 1,2 mm versehen mit einem Rückflusskühler und Magnetrührer wird unter Argon mit 1,3 mg Rh(PMe₃)₂(CO)Cl (0.004 mmol) und 3.1 mg 4,4'-Bipyridin (L2, 0.02 mmol) bestückt. Anschließend wird das Reaktionsgefäß dreimal evakuiert und mit Argon befüllt, um inerte Bedingungen zu realisieren. Es werden anschließend 5 ml n-Octan (30 mmol) zugegeben. Die Rührgeschwindigkeit wird auf 1000 min⁻¹ eingestellt und das Glasgefäß mit Aluminiumfolie umschlossen. Es wird mittels einer Metallkapillare ein CO₂- Strom durch die Lösung geleitet. Dann wird die verwendete Lichtquelle Lumatec Superlite 400 angeschaltet. Nach der Reaktion wird die Lichtquelle ausgeschaltet, der CO₂-Strom beendet, die Reaktionslösung abgekühlt und die Ausbeute mittels Gaschromatografie und Isooctan als internem Standard bestimmt.

**Tabelle 7**

| Eintrag | Zeit (h) | ohne CO₂ | | mit CO₂ | |
|---|---|---|---|---|---|
| | | Ausbeute (%) | TON | Ausbeute (%) | TON |
| 1 | 3 | 11.3 | 850 | 12.3 | 881 |
| 2 | 5 | 16.7 | 1353 | 16.0 | 1197 |
| 3 | 7 | 19.7 | 1518 | 17.0 | 1250 |
| 4 | 14 | 19.6 | 1495 | 23.0 | 1666 |

### Beispiel 8

Reaktion unter Verwendung eines 1,2 mm dicken einwandigen Schlenkgefäßes mit Cycloctan als Substrat, dem Additiv L2 (4,4'-Bipyridin) und [Rh(CO)Cl(PPh₂CH₂PPh₂)]₂ als Katalysator.

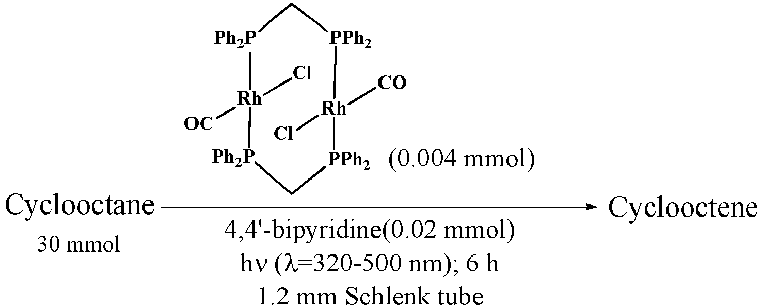

Ein Schlenkgefäß mit einer Wandstärke von 1,2 mm versehen mit einem Rückflusskühler und Magnetrührer wird unter Argon mit 4.4 mg [Rh(CO)Cl(PPh₂CH₂PPh₂)]₂ (0.004 mmol) und 3.1 mg 4,4'-Bipyridin (L2, 0.02 mmol) bestückt. Anschließend wird das Reaktionsgefäß dreimal evakuiert und mit Argon befüllt, um inerte Bedingungen zu realisieren. Es werden anschließend 4 ml Cyclooctan (30 mmol) zugegeben. Es wird ein Argonstrom angelegt, um gebildeten Wasserstoff zu entfernen. Der Reaktor wird mit Aluminiumfolie umschlossen und die Lichtquelle mit einer Wellenlänge 320-500 nm Wellenlänge (Lumatec Superlite 400) angeschaltet. Nach der Reaktion wird die Lichtquelle ausgeschaltet, der Argon-Strom abgeschaltet, die Reaktionslösung abgekühlt und die Ausbeute mittels Gaschromatografie und Isooctan als internem Standard bestimmt.

**Tabelle 8**

| Entry | Zeit (h) | Ausbeute (%) | TON | TOF (h⁻¹) |
|---|---|---|---|---|
| 1 | 6 | 9 | 650 | 108 |

## Patentansprüche

1. Verfahren zur photokatalytischen akzeptorfreien Dehydrierung von Alkanen und Alkoholen,
**dadurch gekennzeichnet, dass** ein Alkan oder ein Alkohol in Gegenwart einer Rhodiumkomplexverbindung, die organische Phosphor(III)-Verbindungen als Liganden enthält, als Katalysator, sowie in Gegenwart von mindestens einer Lewis Base, bestrahlt wird.

2. Verfahren nach Anspruch 1,
wobei die Lewis Base ein organisches Amin ist

3. Verfahren nach einem der Ansprüche 1 oder 2,
wobei die Lewis Base ein heterozyklisches Amin ist.

4. Verfahren nach einem der Ansprüche 1 bis 3,
wobei die Lewis Base ein Bipyridin ist.

5. Verfahren nach einem der Ansprüche 1 bis 4,
wobei als Lewis Base 2,2-Bipyridin oder 4,4-Bipyridin verwendet wird.

6. Verfahren nach einem der Ansprüche 1 bis 5,
wobei als Lewis Base Bathocuproin oder Phenanthrolin verwendet wird.

7. Verfahren nach einem der Ansprüche 1 bis 6,
**dadurch gekennzeichnet, dass** die Reaktion in Gegenwart von CO₂ durchgeführt wird.

8. Verfahren nach einem der Ansprüche 1 bis 7,
**dadurch gekennzeichnet, dass** die Bestrahlung mit Licht einer Wellenlänge von 320 nm bis 500 nm erfolgt.

9. Verfahren nach einem der Ansprüche 1 bis 8,
**dadurch gekennzeichnet, dass** die Dehydrierung bei einer Temperatur von 45 °C bis 120 °C durchgeführt wird.

10. Verfahren nach einem der Ansprüche 1 bis 9,
**dadurch gekennzeichnet, dass** ein Rhodiumkomplex der allgemeinen Formel (I) eingesetzt wird:
Rh(L¹)₂(CO)X **(I),**
worin L¹ = P(C₁-C₅Alkyl)₃ ist,
und X für ein Anion steht ausgewählt aus: Chlorid, Bromid, Acetat.

11. Verfahren nach Anspruch 10,
**dadurch gekennzeichnet, dass** L¹ = PMe₃ oder P*t*Bu₃.

12. Verfahren nach einem der Ansprüche 10 oder 11,
**dadurch gekennzeichnet, dass** X für Chlorid steht.

13. Verfahren nach einem der Ansprüche 1 bis 9,
**dadurch gekennzeichnet, dass** ein Rhodiumkomplex der allgemeinen Formel **(II)** eingesetzt wird:
Rh₂(L²)₂(CO)₂(X)₂ **(II)**,
worin L² = P(PH)₂(CH₂)ₙ(Ph₂)P ist mit n = 1 bis 3,
und X für ein Anion steht ausgewählt aus: Chlorid, Bromid, Acetat.

14. Verfahren nach Anspruch 13,
**dadurch gekennzeichnet, dass** L² = P(PH)₂(CH₂)(Ph₂)P.

15. Verfahren nach einem der Ansprüche 13 oder 14,
**dadurch gekennzeichnet, dass** X für Chlorid steht.

16. Verfahren nach einem der Ansprüche 1 bis 15,
**dadurch gekennzeichnet, dass** das Alkan oder der Alkohol eine Kohlenstoffkettenlänge von 2 bis 30 C-Atome aufweist.

17. Verfahren nach einem der Ansprüche 1 bis 16,
**dadurch gekennzeichnet, dass** der gebildete Wasserstoff aus dem Reaktionsgemisch entfernt wird.
